# EUROPEAN PATENT SPECIFICATION

(11) **EP 4 570 210 B1**
(45) Date of publication and mention of the grant of the patent: **28.10.2026**
(21) Application number: 23851927.6
(22) Date of filing: 10.08.2023
(51) Int. Cl.: G06T 7/33, G06T 7/73, A61B 34/10, A61B 34/20

(54) **REGISTRATION POINT DETERMINATION AND REGISTRATION METHOD AND APPARATUS, DEVICE, MEDIUM, AND PROGRAM PRODUCT**
REGISTRIERUNGSPUNKTBESTIMMUNGS- UND REGISTRIERUNGSVERFAHREN UND -VORRICHTUNG, VORRICHTUNG, MEDIUM UND PROGRAMMPRODUKT
PROCÉDÉ ET APPAREIL DE DÉTERMINATION ET D'ENREGISTREMENT DE POINT D'ENREGISTREMENT, DISPOSITIF, SUPPORT ET PRODUIT DE PROGRAMME

(30) Priority: 12.08.2022 CN 202210968555
(43) Date of publication of application: 18.06.2025
(73) Proprietor: Beijing Hurwa Medical Technology Co., Ltd., Beijing 100176 (CN)
(72) Inventor: DU, Kebin, Beijing 100176 (CN); LIU, Pengchun, Beijing 100176 (CN); JIANG, Biao, Beijing 100176 (CN)
(74) Representative: Murgitroyd & Company
(86) International application number: PCT/CN2023/112183
(87) International publication number: WO 2024/032706

(56) References cited:
- WO-A1-2021/243977
- AU-A1- 2021 286 411
- CN-A- 104 869 934
- CN-A- 108 175 501
- CN-A- 109 464 196
- CN-A- 109 953 828
- CN-A- 114 376 724
- CN-A- 116 725 679
- US-A1- 2014 187 955
- US-A1- 2014 187 955
- US-A1- 2018 101 950
- US-A1- 2018 101 950
- US-A1- 2019 000 561
- US-A1- 2019 192 228
- US-A1- 2021 186 629
- US-A1- 2022 249 169
- ZHAO, YANPENG: "Development of a Surgical Robot System for Long Bone Fractures", MEDICINE AND HEALTH SCIENCES, CHINA DOCTORAL DISSERTATIONS FULL-TEXT DATABASE, 29 May 2016 (2016-05-29), CN, pages 1 - 106, XP009552840

## Description

### TECHNICAL FIELD

The present disclosure relates to the field of data processing technologies, and in particular, to a registration point determination and registration method and apparatus, a device, a medium, and a program product.

### BACKGROUND

Robotic-assisted joint replacement surgery is currently a hot topic in research. A general process of the surgery is as follows. First, in a preoperative phase, three-dimensional reconstruction is performed on medical image data (for example, may be data of Computed Tomography, CT) of a patient, so as to generate a three-dimensional skeleton model of the patient, and a related feature point (specifically, may be a feature point of a joint part to be operated on) in the three-dimensional skeleton model is marked. Based on this, an operation scheme is planned. In an operation process, point collection is performed on a surface of a bone on an operation side of the patient to generate a point cloud, then registration is performed between the acquired point cloud and a preoperative three-dimensional model to find a spatial mapping relationship of between a preoperative plan and a real bone during the surgery, and surgical operations are instructed according to the mapping relationship. It may be seen that accurate collection of points on the bone surface (used for registration) is important in the robotic-assisted joint replacement surgery.

US2018101950A1 discloses to a processor that receives signals from a probe, as the probe is placed at respective locations on a body of a subject, and derives, from the signals, a set of points that correspond to the respective locations. The processor then registers the set of points with a three-dimensional image of the body, by identifying, for each of the points, one or more voxels of the image whose respective surface normals are oriented within a given angle of an orientation at which the probe was held at the location to which the point corresponds, and computing a transformation that aligns each of the points with a selected one of the voxels that were identified for the point. The processor then tracks, based on the registering, a location of an intrabody tool, in a coordinate system of an imaging device that was used to acquire the image.

### SUMMARY

Embodiments of the present disclosure aim to provide a registration point determination and registration method and apparatus, an electronic device, a medium, and a program product, which solves a problem that a signal needs to be transmitted during each manually hovering of a probe to collect the point. Therefore, labor is saved to the maximum extent possible while reducing errors caused by human operations, thereby saving time and effort, improving collection speed and convenience, and achieving high collection efficiency.

Technical solutions in the present disclosure are as follows.

According to a first aspect, a registration point determination method is provided. The method includes: acquiring a first pose data set of a probe when a collection signal is received; determining a hovering coordinate set of the probe based on the first pose data set of the probe, wherein the hovering coordinate set is a set of coordinate data of the probe that meets a hover condition, wherein the hover condition is a preset condition for determining that the probe is in a hover state; and determining, based on the hovering coordinate set, a registration point corresponding to the first pose data set of the probe, where the determining, based on the hovering coordinate set, a registration point corresponding to the first pose data set of the probe, includes: determining, based on the hovering coordinate set, average coordinates of coordinates corresponding to all elements in the hovering coordinate set; and determining a point corresponding to the average coordinates of the coordinates corresponding to all elements in the hovering coordinate set as the registration point corresponding to the first pose data set of the probe.

In some embodiments of the present disclosure, the acquiring a first pose data set of a probe when a collection signal is received includes: acquiring the first pose data set of the probe and a second pose data set of a reference object when the collection signal is received, where the determining a hovering coordinate set of the probe based on the first pose data set of the probe includes: determining, based on the first pose data set and the second pose data set, a relative coordinate data set of the probe relative to the reference object; and determining the hovering coordinate set of the probe based on the relative coordinate data set.

In some embodiments of the present disclosure, the determining a hovering coordinate set of the probe based on the first pose data set of the probe includes: determining a check data set based on the first pose data set of the probe; and determining the hovering coordinate set of the probe based on the first pose data set of the probe and the check data set.

In some embodiments of the present disclosure, the determining a check data set based on the first pose data set of the probe includes: determining an A^{th} element in the check data set, where the determination is as follows: calculating an average value of top A elements in the first pose data set of the probe, and determining the average value as the A^{th} element in the check data set to obtain the check data set, where A is a positive integer.

In some embodiments of the present disclosure, the determining the hovering coordinate set of the probe based on the first pose data set of the probe and the check data set includes: determining a first element in the first pose data set of the probe as a first element in the hovering coordinate set of the probe; and determining a B^{th} element in the hovering coordinate set, where the determination is as follows: in a case that a distance between coordinates corresponding to a (B-1)^{th} element in the check data set and coordinates corresponding to a B^{th} element in the first pose data set of the probe meets a hover condition, determining the B^{th} element in the first pose data set of the probe as the B^{th} element in the hovering coordinate set; and in a case where the distance between coordinates corresponding to a (B-1)^{th} element in the check data set and coordinates corresponding to a B^{th} element in the first pose data set of the probe fails to meet the hover condition, determining that the hovering coordinate set includes only B-1 elements, wherein B is a positive integer, and B ≥ 2.

In some embodiments of the present disclosure, the determining, based on the hovering coordinate set, a registration point corresponding to the first pose data set of the probe includes: when a quantity of elements in the hovering coordinate set meets a preset quantity threshold, determining coordinates corresponding to the last element in the hovering coordinate set; determining average coordinates of coordinates corresponding to all elements in the hovering coordinate set; when a distance between the coordinates corresponding to the last element and the average coordinates meets a registration point condition, determining that there is a registration point in the hovering coordinate set; and determining, based on the hovering coordinate set, the registration point corresponding to the first pose data set of the probe.

In some embodiments of the present disclosure, the acquiring a first pose data set of a probe when a collection signal is received includes: acquiring the first pose data set of the probe within a preset time period when the collection signal is received.

In some embodiments of the present disclosure, the acquiring a first pose data set of a probe when a collection signal is received includes: acquiring the first pose data set of the probe during a period from a start to an end of the collection signal when the collection signal is received.

In some embodiments of the present disclosure, the first pose data set includes a first data enable bit, a first data frame sequence, and first time stamp information, and the second pose data set includes a second data enable bit, a second data frame sequence, and second time stamp information; and the determining, based on the first pose data set and the second pose data set, a relative coordinate data set of the probe relative to the reference object includes: determining, based on the first pose data set and the second pose data set, the relative coordinate data set of the probe relative to the reference object when the first data enable bit and the second data enable bit are preset data enable bits, the first data frame sequence is the same as the second data frame sequence, and the first time stamp information is the same as the second time stamp information.

According to a second aspect, a registration method is provided. A registration point in the method is determined based on the registration point determination method according to the first aspect.

According to a third aspect, a registration point determination apparatus is provided. The apparatus includes: a first acquisition module, configured to acquire a first pose data set of a probe when a collection signal is received; a first determination module, configured to determine a hovering coordinate set of the probe based on the first pose data set of the probe, wherein the hovering coordinate set is a set of coordinate data of the probe that meets a hover condition, wherein the hover condition is a preset condition for determining that the probe is in a hover state ; and a second determination module, configured to determine, based on the hovering coordinate set, a registration point corresponding to the first pose data set of the probe, where the second determination module (430) is further configured to: determine, based on the hovering coordinate set, average coordinates of coordinates corresponding to all elements in the hovering coordinate set; and determine a point corresponding to the average coordinates of the coordinates corresponding to all elements in the hovering coordinate set as the registration point corresponding to the first pose data set of the probe.

According to a fourth aspect, an embodiment of the present disclosure provides an electronic device. The electronic device includes a processor, a memory, and a program or an instruction that is stored in the memory and executable on the processor. The program or instruction is executed by the processor to implement the steps of the registration point determination method according to any one of embodiments of the present disclosure.

According to a fifth aspect, an embodiment of the present disclosure provides a readable storage medium. The readable storage medium has a program or an instruction stored thereon. The program or instruction, when executed by a processor, implements the steps of the registration point determination method according to any one of embodiments of the present disclosure.

According to a sixth aspect, an embodiment of the present disclosure provides a computer program product. An instruction in the computer program product, when executed by a processor of an electronic device, causes the electronic device to execute the steps of the registration point determination method according to any one of embodiments of the present disclosure.

Beneficial effects at least brought by the technical solutions provided in the embodiments of the present disclosure are as follows.

In the embodiments of the present disclosure, when the collection signal is received, the hovering coordinate set of the probe may be determined based on the acquired first pose data set of the probe, and the registration point corresponding to the first pose data set of the probe may be accurately determined based on the hovering coordinate set. Thus, a set including at least one piece of pose data of the probe, that is, the first pose data set of the probe, is acquired at a time, data that meets the hover condition is determined from the first pose data set to determine the hovering coordinate set, and then data that may be used as the registration point is determined from the first pose data set of the probe based on the hovering coordinate set, which solves a problem that a signal needs to be transmitted during each manually hovering of a probe to collect the point. Therefore, labor is saved to the maximum extent possible while reducing errors caused by human operations, thereby saving time and effort, improving collection speed and convenience, and achieving high collection efficiency.

It should be understood that the foregoing general description and the following detailed description are merely illustrative and explanative, and are not intended to limit the present disclosure.

### BRIEF DESCRIPTION OF THE DRAWINGS

The accompanying drawings herein, which are incorporated into and form a part of the description, illustrate the embodiments in line with the present disclosure and are used in conjunction with the description to explain a principle of the present disclosure, and do not constitute an improper limitation on the present disclosure.
FIG. 1 is a flowchart of a registration point determination method according to an embodiment in the first aspect of the present disclosure.
FIG. 2 is a schematic diagram of a probe and reference frames according to an embodiment in the first aspect of the present disclosure.
FIG. 3 is a flowchart of another registration point determination method according to an embodiment in the first aspect of the present disclosure.
FIG. 4 is a schematic structural diagram of a registration point determination apparatus according to an embodiment in the third aspect of the present disclosure.
FIG. 5 is a schematic structural diagram of an electronic device according to an embodiment in the fourth aspect of the present disclosure.

### DETAILED DESCRIPTION OF THE EMBODIMENTS

Technical solutions in the embodiments of the present disclosure will be described clearly and completely below with reference to accompanying drawings in the embodiments of the present disclosure, so that those skilled in the art may understand the technical solutions in the present disclosure better. It should be understood that specific embodiments described herein are merely intended to explain the present disclosure, and are not intended to limit the present disclosure. For those skilled in the art, the present disclosure may be implemented in a case where some of these specific details are not required. The following description of the embodiments is merely used to provide a better understanding of the present disclosure by illustrating examples of the present disclosure.

It should be noted that, in the specification, claims, and accompanying drawings of the present disclosure, terms such as "first" and "second" are intended to distinguish between similar objects but do not necessarily indicate a specific order or sequence. It should be understood that the data used in this way may be interchangeable under appropriate circumstances such that embodiments in the present disclosure described herein are capable of being implemented in an order different from that illustrated or described herein. The implementations described in the following exemplary embodiments do not represent all implementations consistent with the present disclosure. Instead, they are only examples that are described in the appended claims in detail and that are consistent with some aspects of the present disclosure.

During collection of a registration point, a probe needs to be placed at a corresponding position, then a pedal is stepped on or a confirm button is clicked in software to set this point as the registration point, and a position of this point is acquired through a navigator. In view of this, when a large quantity of registration points needs to be collected, an acknowledgment signal needs to be transmitted during collection of each point, thereby undoubtedly consuming unnecessary time and effort of an operator. In addition, as the acknowledgment signal needs to be transmitted when collecting the registration point, in a process of a large quantity of repeated operations (for example, for each collection point, the probe needs to be manually held to collect the point, and the pedal needs to be stepped on for conformation, requiring repeated use of both hand and feet), it is easy for the operator to make mistakes.

During collection of the plurality of registration points, repeated operations may consume unnecessary time and effort, resulting in low collection efficiency. To resolve the foregoing problems, embodiments of the present disclosure provide a registration point determination method and apparatus, an electronic device, a medium, and a program product. When a collection signal is received, a hovering coordinate set of a probe may be determined based on an acquired first pose data set of the probe. A registration point corresponding to the first pose data set of the probe may be accurately determined based on the hovering coordinate set. In this way, a set including at least one piece of pose data of the probe, that is, the first pose data set of the probe, is acquired at a time, and then data that meets a hover condition is determined from the first pose data set to determine the hovering coordinate set. Then, data that may be used as the registration point is determined from the first pose data set of the probe based on the hovering coordinate set, which solves a problem that a signal needs to be transmitted during each manually hovering of a probe to collect the point. Therefore, labor is saved to the maximum extent possible while reducing errors caused by human operations, thereby saving time and effort, improving collection speed and convenience, and achieving high collection efficiency.

With reference to the accompanying drawings, the registration point determination method provided by the embodiments of the present disclosure will be described through specific embodiments and application scenarios thereof.

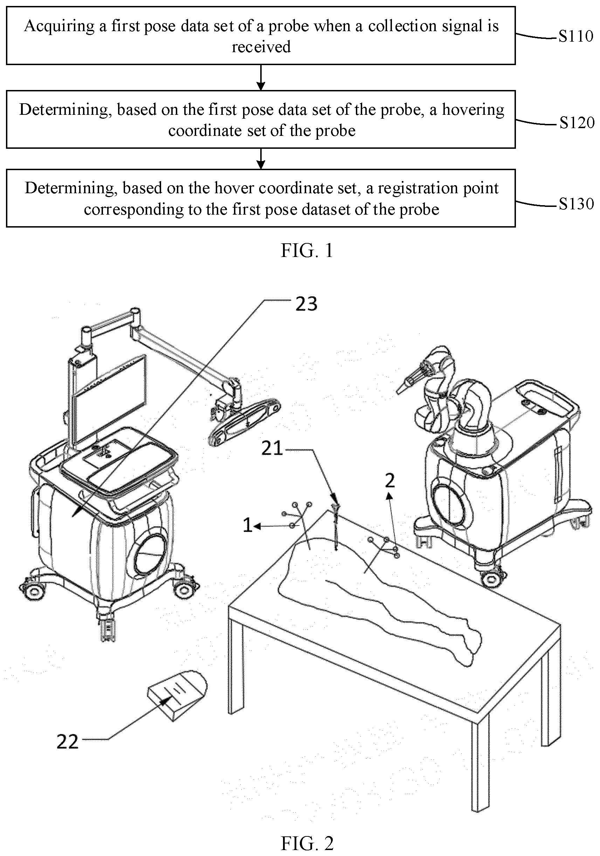

FIG. 1 is a flowchart of a registration point determination method according to an embodiment of the present disclosure. An execution body of the registration point determination method may be a server. It should be noted that the execution body does not constitute a limitation on the present disclosure.

As shown in FIG. 1, the registration point determination method provided by the embodiment of the present disclosure may include steps S110 to S130.

Step S110: acquiring a first pose data set of a probe when a collection signal is received.

The collection signal may be a signal to collect data. The collection signal may be triggered and generated in response to a signal collection operation of a user, for example, may be generated by stepping on a pedal by the user.

In some embodiments of the present disclosure, the collection signal may alternatively be generated based on another trigger operation. For example, the collection signal may be triggered by clicking a signal collection button on a software interface by a user, or through voice control or gesture control, which is not limited herein.

The first pose data set may be a pose data set of a probe used for collection. For example, a set of pose data of the probe is collected within a preset time period (for example, 500 milliseconds), or a set of pose data of the probe is collected in preset storage space (a set of pose data of the probe obtained when the collected data fills the storage space). The first pose data set is a set including at least one piece of pose data of the probe. Optionally, the first pose data set may be a set of a plurality of pieces of pose data of the probe.

In some embodiments of the present disclosure, to accurately acquire the first pose data set, step S110 may specifically include the flollowing step.

Step S1101: acquiring the first pose data set of the probe during a period from a start to an end of the collection signal when the collection signal is received.

In an embodiment, taking that the collection signal is generated by stepping on the pedal by the user as an example, the start of the collection signal is when the user steps on the pedal, and the end of the collection signal is when the user releases the pedal. If the user keeps stepping on the pedal, all pose data of the probe is acquired to form the first pose data set. The first pose data set may include pose data of the probe when it is moved by the user, and pose data of the probe when movement of the probe is stopped by the user.

In this embodiment of the present disclosure, the first pose data set of the probe during the period from the start to the end of the collection signal is acquired when the collection signal is received. Thus, a set including at least one piece of pose data of the probe is acquired at a time, and there is no need for neither a plurality of times of acquisition nor a plurality of operations, so that labor is saved to improve convenience.

In some embodiments of the present disclosure, step S110 may include the flollowing step.

Step S1102: acquiring the first pose data set of the probe within a preset time period when the collection signal is received.

The preset time period may be a time interval set in advance, for example, may be 10 milliseconds. For example, in a case where the collection signal is a continuous signal, the first pose data set of the probe is continuously acquired, so that effects of continuous collection and cyclic collection may be achieved.

Step S120: determining a hovering coordinate set of the probe based on the first pose data set of the probe.

The hovering coordinate set may be a set of coordinate data of the probe that meets a hover condition.

In some embodiments of the present disclosure, to accurately determine the hovering coordinate set, step S110 may include the following step.

Step S1103: acquiring the first pose data set of the probe and a second pose data set of a reference object when the collection signal is received.

Accordingly, step S120 may specifically include the following steps.

Step S12031: determining, based on the first pose data set and the second pose data set, relative coordinate data set of the probe relative to the reference object.

Step S12032: determining the hovering coordinate set of the probe based on the relative coordinate data set.

The reference object may be selected from objects that are stationary and whose pose data is known. For accuracy, pose data of the reference object that is obtained synchronously with the probe may be used. It may be understood that, for data accuracy, a stationary object is selected as the reference object. It may also be understood that, to reduce occurrence of accidental collisions, an object near a collection point is selected as the reference object; or an object with a positioning apparatus is used as the reference object, and the object is fixed to an object where the collection point is located.

The second pose data set may be a pose data set of the reference object that is collected. It should be noted that the first and the second herein are used to distinguish between the probe and the reference object, and this does not mean that there is only one pose in the data set. Thus, the hovering coordinate set is determined based on the relative coordinate data set. Relative coordinate data or first pose data corresponding to the relative coordinate data may be stored in the hovering coordinate set. If the relative coordinate data is stored in the hovering coordinate set, the first pose data may also be determined from the relative coordinate data.

Any pose data set in the first pose data set and the second pose data set may include a spatial posture (represented in a quaternion form: w, x, y, and z, where w denotes an angle, and x, y, and z denote coordinate values), three-dimensional coordinates (x, y, and z), a data enable bit (used to indicate whether the data is valid, specifically, if the enable bit is "1", it indicates that the data is valid; and if the enable bit is "0", it indicates that the data is invalid), a data frame count, a time stamp, and other data.

In some embodiments of the present disclosure, since the reference object may be selected from objects that are stationary and whose pose data is known, the second pose data set of the reference object in step S 110 may be data of only one pose. When the relative coordinate data set of the probe relative to the reference object is determined in step S120, the relative coordinate data set may be determined based on the only one pose and the first pose data set. In some embodiments of the present disclosure, alternatively, the second pose data set of the reference object in step S 110 is data of at least one pose. When the relative coordinate data set of the probe relative to the reference object is determined in step S 120, a piece of data is selected from data of the at least one pose or an average value of the data of the at least one pose is calculated, and the relative coordinate data set is determined based on the first pose data set and the piece of data or the average value.

In some embodiments of the present disclosure, the navigator continuously collects the first pose data of the probe and the second pose data of the reference object, and then stores the first pose data of the probe and the second pose data of the reference object. When it is detected that the pedal is stepped on, a system starts to acquire the first pose data set and the second pose data set collected by the navigator.

In some embodiments of the present disclosure, acquisition and storage of the first pose data set and the second pose data set are asynchronous. In the following, asynchronous acquisition and storage of the pose data in the first pose data set and the second pose data set may be described.

If the navigator outputs pose information (the first pose data set and the second pose data set) at a frequency of 60 Hz, these pose information is stored and overwritten in real time. Data of a current frame overwrites data of a previous frame. Therefore, only data of the current frame is retained during storage, and data of the current frame is continuously refreshed. During acquisition (when the pedal is stepped on), information of the current frame is extracted from the stored pose information and applied to subsequent calculation for use whenever required. In other words, the stored pose information is continuously refreshed, and starts to be acquired according to a step-on signal of pedal during acquisition, or is acquired within a required time period, so as to implement asynchronous storage and acquisition. There is no need for acquisition and calculation immediately after a group of information is stored, thereby improving efficiency and reducing calculation amount.

In an embodiment, as shown in FIG. 2, during a hip joint replacement surgery, a hip bone reference frame (as marked by reference numeral of 1 shown in FIG. 2) is placed in a proper position on a surface of a hip bone while performing acetabular reaming or acetabular cup prosthesis placement. During femoral osteotomy, a femur reference frame (as marked by reference numeral of 2 shown in FIG. 2) is placed in a proper position on a surface of the femur. Therefore, in different phases, the hip bone reference frame or the femur reference frame may be selected as the reference object according to requirements. A probe 21 is used to collect a registration point. After a pedal 22 is stepped on, the system starts to acquire the pose data of the probe and the pose data of the reference frame that are collected by the navigator. It should be noted that in FIG. 2, a processing module and a storage module configured to implement the solution are equipped in a console vehicle 23.

The relative coordinate data set is a coordinate data set of the probe relative to the reference object. Specifically, a relative vector between coordinates of the probe and coordinates of the reference object may be first calculated, then a quaternion of the reference object is normalized and inverted, and the relative vector is rotated according to the inverted quaternion of the reference object to obtain the relative coordinate data set of the probe relative to the reference object.

In an embodiment, for example, if the first pose data of the probe is A and the second pose data of the reference object is B, the relative coordinate data set of the probe relative to the reference object may be A-B.

It should be noted that the reference object in the relative coordinate data set of the probe relative to the reference object is one of the reference frames in FIG. 2, and is not two reference frames. Specifically, a reference object for which calculation is to be performed may be determined based on a skeleton type of a registration point to be collected.

In an embodiment, if a knee replacement surgery is to be performed in this case, a reference frame needs to be placed on each of the femur and the tibia. When collecting pose data, the navigator collects three types of pose data: pose data of the probe, pose data of the femur reference frame, and pose data of the tibia reference frame. However, during collection of a registration point on a part, for example, during collection of a registration point on the femur (a bone type), only the pose data of the probe and the pose data of the femur reference frame need to be considered. Therefore, the pose data of the tibia reference frame is filtered out, and only the pose data of the probe and the pose data of the femur reference frame are acquired.

In an embodiment, if a hip joint replacement surgery is to be performed in this case, a reference frame needs to be placed on each of the femur and the hip bone. When collecting pose data, the navigator collects three types of pose data: pose data of the probe, pose data of the femur reference frame, and pose data of the hip bone reference frame. However, during collection of a registration point on a part, for example, during collection of a registration point on the femur (a bone type), only the pose data of the probe and the pose data of the femur reference frame need to be considered. Therefore, the pose data of the hip bone reference frame is filtered out, and only the pose data of the probe and the pose data of the femur reference frame are acquired.

By using the relative coordinate data set in this way, a problem of a deviation in captured pose data that is caused by an accidental contact with the navigator during the surgery may be avoided.

In this embodiment of the present disclosure, when the collection signal is received, the first pose data set of the probe and the second pose data set of the reference object are acquired. Then, the relative coordinate data set of the probe relative to the reference object is determined based on the first pose data set and the second pose data set, and the hovering coordinate set of the probe is determined based on the relative coordinate data set. Thus, consistency of real-time pose data between the probe and the reference object may be ensured, and the coordinates, relative to the coordinates of the reference object, of the probe are stored, which may avoid a problem of a deviation in captured pose data that is caused by an accidental contact with the navigator during the surgery.

In some embodiments of the present disclosure, to improve registration accuracy, the first pose data set may include a first data enable bit, a first data frame sequence, and first time stamp information, and the second pose data set may include a second data enable bit, a second data frame sequence, and second time stamp information. Step S12031 of determining, based on the first pose data set and the second pose data set, a relative coordinate data set of the probe relative to the reference object may specifically include the following step.

Step S120311: determining, based on the first pose data set and the second pose data set, the relative coordinate data set of the probe relative to the reference object when the first data enable bit and the second data enable bit are preset data enable bits, the first data frame sequence is the same as the second data frame sequence, and the first time stamp information is the same as the second time stamp information.

The first data enable bit may be a data enable bit in the pose data of the probe. The first data frame sequence may be a collected frame sequence used to represent the pose data of the probe. The first time stamp information may be time information of collection used to represent the pose data of the probe. The second data enable bit may be a data enable bit used to represent the pose data of the reference object. The second data frame sequence may be a collected frame sequence used to represent the pose data of the reference object. The second time stamp information may be time information of collection used to represent the pose data of the reference object. The preset data enable bit may be a data enable bit set in advance, and specifically, may be an enable bit used to represent that the pose data is valid data, for example, may be "1".

In this embodiment of the present disclosure, by acquiring the first data enable bit, the first data frame sequence, and the first time stamp information in the first pose data, and the second data enable bit, the second data frame sequence, and the second time stamp information in the second pose data, when the first data enable bit and the second data enable bit are preset data enable bits, the first data frame sequence is the same as the second data frame sequence, and the first time stamp information is the same as the second time stamp information, the relative coordinate data set of the probe relative to the reference object is determined based on the first pose data set and the second pose data set. Thus, temporal consistency between the pose data of the probe and the pose data of the reference object, as well as validity of the pose data of the probe and the pose data of the reference object are ensured, so that accuracy of registration point determination is further improved.

**In** some embodiments of the present disclosure, to accurately determine a hover position of the probe, step S120 may specifically include the following steps.

Step S1201: determining a check data set based on the first pose data set of the probe.

Step S1202: determining the hovering coordinate set of the probe based on the first pose data set of the probe and the check data set.

The check data set may be a data set determined based on the first pose data set of the probe. It may be understood that, the check data set is an intermediate data set for determining whether data in the first pose data set meets a hover condition.

**In** this embodiment of the present disclosure, the check data set may be determined based on the first pose data set of the probe, and then the hovering coordinate set of the probe may be determined based on the first pose data set of the probe and the check data set. Thus, by determining the hovering coordinate set is determined based on the check data set and the first pose data set, hover data in the first pose data set may be accurately determined, and the registration point may be further accurately determined.

Similarly, if the first pose data set in this embodiment of the present disclosure is replaced with the relative coordinate data set of the probe relative to the reference object, the foregoing steps may also be implemented and the foregoing effects may also be achieved.

**In** some embodiments of the present disclosure, step S120 may further specifically include the following steps.

Step S1201: determining a check data set based on the first pose data set of the probe.

Step S1203: determining the hovering coordinate set of the probe based on the check data set.

**In** an embodiment, if the first pose data set is (x1, x2, x3, ..., xn), the check data set may be (0, |x1-x2|, |x2-x3|, ..., |x (n-1)-xn|). **If** an element in the check data set meets a preset check threshold, the element may be determined as an element in the hovering coordinate set of the probe.

Similarly, if the first pose data set in this embodiment of the present disclosure is replaced with the relative coordinate data set of the probe relative to the reference object, the foregoing steps may also be implemented and the foregoing effects may also be achieved.

**In** some embodiments of the present disclosure, to further accurately determine the hover position of the probe, the step S1201 of determining a check data set based on the first pose data set of the probe may include the following step.

Step S12011: determining an A^{th} element in the check data set, where the determination is as follows: calculating an average value of top A elements in the first pose data set of the probe, and determining that the average value is the A^{th} element in the check data set to obtain the check data set, where A is a positive integer.

In an example, if the first pose data set is (x1, x2, x3, x4, ..., xn), the check data set may be (x1, (x1+x2)/2, (x1+x2+x3)/3, (x1+x2+x3+x4)/4, ..., (x1+x2+x3+x4+ ... +xn)/n). It should be noted that xn (n is a positive integer) is used herein to represent one piece of coordinate data, and the coordinate data may actually be three-dimensional Cartesian coordinates such as (an, bn, cn).

In this embodiment of the present disclosure, by calculating the average value of the top A elements in the first pose data set of the probe, the A^{th} element in the check data set is determined. Thus, the check data set is obtained, and an accurate hovering coordinate set of the probe may be obtained based on the check data set. Therefore, the hover position of the probe may be accurately determined, and the registration point may be further accurately determined.

Similarly, if the first pose data set in this embodiment of the present disclosure is replaced with the relative coordinate data set of the probe relative to the reference object, the foregoing steps may also be implemented and the foregoing effects may also be achieved.

In some embodiments of the present disclosure, to further accurately determine the hover position of the probe, the step S1202 of determining the hovering coordinate set of the probe based on the first pose data set of the probe and the check data set includes the following step.

Step S12021: determining a first element in the first pose data set of the probe as the first element in the hovering coordinate set of the probe.

Step S12022: determining a B^{th} element in the hovering coordinate set, where the determination is as follows: when a distance between coordinates corresponding to a (B-1)^{th} element in the check data set and coordinates corresponding to a B^{th} element in the first pose data set of the probe meets a hover condition, determining the B^{th} element in the first pose data set of the probe as the B^{th} element in the hovering coordinate set; or when the distance between the coordinates corresponding to a (B-1)^{th} element in the check data set and the coordinates corresponding to a B^{th} element in the first pose data set of the probe the hover condition fails to meet the hover condition, determining that the hovering coordinate set includes only B-1 elements, where B is a positive integer, and B ≥ 2.

The hover condition may be a preset condition for determining that the probe is in a hover state. Specifically, the hover condition may configured to be that the distance between the coordinates corresponding to the (B-1)^{th} element in the check data set and the coordinates corresponding to the B^{th} element in the first pose data set of the probe is less than a preset threshold (for example, may be 1 millimeter).

It should be noted that, it is determined that the first element in the first pose data set of the probe is the first element in the hovering coordinate set of the probe, where the determination of the B^{th} element in hovering coordinate set is as follows: when a distance between coordinates corresponding to a (B-1)^{th} element in the check data set and coordinates corresponding to a B^{th} element in the first pose data set of the probe meets a hover condition, determining the B^{th} element in the first pose data set of the probe as the B^{th} element in the hovering coordinate set; or when the distance between the coordinates corresponding to a (B-1)^{th} element in the check data set and the coordinates corresponding to a B^{th} element in the first pose data set of the probe the hover condition fails to meet the hover condition, determining that the hovering coordinate set includes only B-1 elements, where B is a positive integer, and B ≥ 2.

In an embodiment, if the first pose data set is (x1, x2, x3, x4, ..., xn), and the check data set is (x1, (x1+x2)/2, (x1+x2+x3)/3, (x1+x2+x3+x4)/4, ..., (x1+x2+x3+x4+ ... +xn)/n), the first element x1 in the first pose data set may be determined as the first element in the hovering coordinate set of the probe. Next, other elements in the hovering coordinate set may be determined. For the second element in the hovering coordinate set, in this case, B is 2. If a distance between coordinates (that is, x1) corresponding to the first (that is, (B-1^{th}) element in the check data set and coordinates (that is, x2) corresponding to the second (that is, (B^{th})) element in the first pose data set meets the hover condition, the second (that is, B^{th}) element in the first pose data set is determined as the second (that is, B^{th}) element in the hovering coordinate set. Then, the third element in the hovering coordinate set is considered, that is, B is 3, and details are not described herein. If the distance between the coordinates (that is, x1) corresponding to the first (that is, (B-1)^{th} element in the check data set and the coordinates (that is, x2) corresponding to the second (that is, B^{th}) element in the first pose data set fails to meet the hover condition, the hovering coordinate set is (x1).

In this embodiment of the present disclosure, the distance between the coordinates corresponding to the (B-1)^{th} element in the check data set and the coordinates corresponding to the B^{th} element in the first pose data set is determined to determine which elements in the first pose data set may be used as elements in the hovering coordinate set. Thus, when an element fails to meet the hover condition, elements after the element are no longer considered, which reduces excessive calculation time, and thereby quickly and efficiently determining the hovering coordinate set.

Similarly, if the first pose data set in this embodiment of the present disclosure is replaced with the relative coordinate data set of the probe relative to the reference object, the foregoing steps may also be implemented and the foregoing effects may also be achieved.

Step S130: determining, based on the hovering coordinate set, a registration point corresponding to the first pose data set of the probe.

In some embodiments of the present disclosure, points in the hovering coordinate set are determined to be points collected when the probe is in a hovering state. However, whether these points may be used as the registration point needs to be further determined. A determination method is as follows.

In some embodiments of the present disclosure, to accurately determine a registration point, before step S130, the foregoing registration point determination method may further include the following steps.

Step S125: when a quantity of elements in the hovering coordinate set meets a preset quantity threshold, determining coordinates corresponding to a last element in the hovering coordinate set.

Step S126: determining average coordinates of coordinates corresponding to all elements in the hovering coordinate set.

Step S127: when a distance between the coordinates corresponding to the last element and the average coordinates meets a registration point condition, determining that there is a registration point in the hovering coordinate set.

Specifically, step S130 may include the following step.

Step S1301: determining, based on the hovering coordinate set, the registration point corresponding to the first pose data set of the probe when there is a registration point in the hovering coordinate set.

The preset quantity threshold may be a preset threshold of a quantity of elements in the hovering coordinate set. Specifically, the preset quantity threshold may be a maximum amount of data that can be stored in the hovering coordinate set. The registration point condition may be a preset condition for determination of a registration point. For example, the preset condition may be configured to be that the distance between the coordinates corresponding to the last element and the average coordinates us less than 1 millimeter. It may be understood that, the registration point corresponding to the first pose data set of the probe is determined based on the hovering coordinate set, where data in the hovering coordinate set may include a plurality of pieces of hover pose data, and the determined registration point may include a plurality of registration points that can be used for registration in the plurality of pieces of hover pose data.

In an embodiment, if the hovering coordinate set is (x1, x3, x4) and the preset quantity threshold is 3, the coordinates corresponding to the last element x4 in the hovering coordinate set are obtained, and then the coordinates of x4 are compared with average coordinates of (x1, x3, x4). If a distance between the coordinates of x4 and the average coordinates of (x1, x3, x4) is less than 1 millimeter, it is determined that there is a registration point that can be used for registration in the hovering coordinate set, and then a target registration point that is finally to be used for registration may be determined based on these registration points that can be used for registration.

In this embodiment of the present disclosure, if the quantity of elements in the hovering coordinate set meets the preset quantity threshold, the coordinates corresponding to the last element in the hovering coordinate set are determined. And the average coordinates of the coordinates corresponding to all elements in the hovering coordinate set are determined. If the distance between the coordinates corresponding to the last element and the average coordinates meets the registration point condition, there is a registration point in the hovering coordinate set. When there is a registration point in the hovering coordinate set, the registration point is determined based on the hovering coordinate set. Thus, the registration point corresponding to the first pose data set of the probe that is determined may be more accurate.

In some embodiments of the present disclosure, before step S130, the registration point determination method may further include the following steps.

Step S125: when a quantity of elements in the hovering coordinate set meets a preset quantity threshold, determining coordinates corresponding to a last element in the hovering coordinate set.

Step S126: determining average coordinates of coordinates corresponding to all elements in the hovering coordinate set.

Step S128: when a distance between the coordinates corresponding to the last element and the average coordinates fails to meet a registration point condition, determining that there is no registration point in the hovering coordinate set.

Specifically, step S130 may include the following step.

Step S1302: discarding the first pose data set of the probe to stop subsequent calculation when there is no registration point in the hovering coordinate set.

In this embodiment of the present disclosure, when the quantity of elements in the hovering coordinate set meets the preset quantity threshold, the coordinates corresponding to the last element in the hovering coordinate set are determined. The average coordinates of the coordinates corresponding to all elements in the hovering coordinate set are determined. When the distance between the coordinates corresponding to the last element and the average coordinates fails to meet the registration point condition, the first pose data set of the probe is discarded, and no subsequent calculation needs to be performed. Thus, data that does not meet the registration point condition is filtered out, thereby reducing a calculation amount while making the determined registration point more accurate.

In some embodiments of the present disclosure, the step S130 of determining, based on the hovering coordinate set, the registration point corresponding to the first pose data set of the probe may include the following steps.

Step S1303: determining, based on the hovering coordinate set, average coordinates of coordinates corresponding to all elements in the hovering coordinate set.

Step S1304: determining a point corresponding to the average coordinates of the coordinates corresponding to all elements in the hovering coordinate set as the registration point corresponding to the first pose data set of the probe.

In this embodiment of the present disclosure, the point corresponding to the average coordinates of the coordinates corresponding to all elements in the hovering coordinate set may be determined as the registration point corresponding to the first pose data set of the probe. **In** this way, an accurate registration point corresponding to the first pose data set of the probe may be obtained.

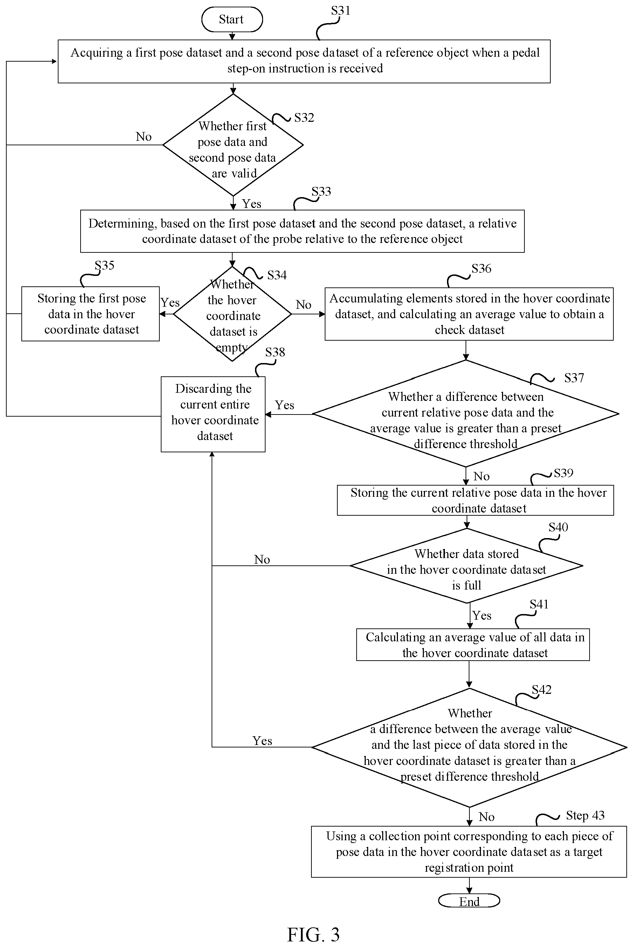

**In** some embodiments of the present disclosure, to understand the technical solutions in the present disclosure more clearly, embodiments of the present disclosure further provide another implementation of the registration point determination method. FIG. 3 is a flowchart of another registration point determination method according to an embodiment of the present disclosure. As shown in FIG. 3, the registration point determination method provided in this embodiment of the present disclosure may include steps S31 to S43.

Step S31: acquiring a first pose data set of a probe and a second pose data set of a reference object when a pedal step-on instruction is received.

This step is consistent with step S1103 of acquiring the first pose data set and the second pose data set in the foregoing embodiment. For brevity, details are not described herein again.

Step S32: determining whether first pose data and second pose data are valid; and if the data is valid, performing step S33; or if the data is invalid, performing step S31.

Validity determination is performed to ensure data validity, such as ensuring temporal consistency between the data of the probe and that of the reference object. Specifically, validity determination includes: determining whether a first data enable bit and a second data enable bit are preset data enable bits, a first data frame sequence is the same as a second data frame sequence, and first time stamp information is the same as second time stamp information.

Step S33: determining, based on the first pose data set and the second pose data set, a relative coordinate data set of the probe relative to the reference object.

This step is consistent with the method of determining the relative coordinate data set in the foregoing embodiment. For brevity, details are not described herein again.

Step S34: determining whether a hover coordinate data set is empty; and if the hover coordinate data set is empty, performing step S35; or if the hover coordinate data set is not empty, performing step S36.

Step S35: storing the first pose data in the hover coordinate data set.

After first pose data is stored in the hover coordinate data set, step S31 may be performed after a preset time period.

Step S36: accumulating elements stored in the hover coordinate data set, and calculating an average value to obtain a check data set.

Step S37: determining whether a difference between current relative pose data and the average value is greater than a preset difference threshold; and if yes, performing step S38; or if no, performing step S39.

Step S38: discarding the current entire hover coordinate data set, and returning to step S31.

Step S39: storing the current relative pose data in the hover coordinate data set.

Step S40: determining whether data in the hover coordinate data set is full; and if the data in the hover coordinate data set is not full, returning to step S31 after a preset time period; or if the data in the hover coordinate data set is full, performing step S41.

Step S41: calculating an average value of all data in the hover coordinate data set.

Step S42: determining whether a difference between the average value and the last piece of relative pose data stored in the hover coordinate data set is greater than a preset difference threshold; and if yes, performing step S38; or if no, performing step S43.

Step S43: determining a collection point corresponding to each piece of pose data in the hover coordinate data set as a target registration point.

The foregoing technical solution may have following several replacement solutions:
(1) When the pedal is stepped on, a collection signal is triggered, current coordinates of the probe are collected to generate a collection point, and the current collection process ends.

In this replacement solution, after the pedal is stepped on, the coordinates of the probe are acquired, the coordinates of the probe are directly determined as a collected registration point, that is, the collected coordinates are directly determined as a target registration point, and the collection ends.

(2) When the pedal is stepped on, a collection signal is triggered, current coordinates of the probe are collected to generate a collection point till a pedal release signal is received, and the current collection process ends.

In this replacement solution, after the pedal is stepped on, the coordinates of the probe are acquired, the coordinates of the probe are directly determined as a collected registration point, that is, the collected coordinates are directly used as a target registration point, and the collection continues to be performed till the pedal is released.

(3) When the pedal is stepped on, a collection signal is triggered, current coordinates of the probe are collected, the current coordinates of the probe are transformed into relative coordinates relative to the reference object to generate a collection point, and the current collection process ends.

In this replacement solution, after the pedal is stepped on, the coordinates of the probe are acquired, the coordinates of the probe are transformed into coordinates relative to the reference object, the coordinates relative to reference object transformed from the coordinated of the probe are directly used as a target registration point, and the collection ends.

(4) When the pedal is stepped on, a collection signal is triggered, current coordinates of the probe are collected, the current coordinates of the probe are transformed into relative coordinates relative to the reference object to generate a collection point, and the current collection process is continued till a pedal release signal is received.

In this replacement solution, after the pedal is stepped on, the coordinates of the probe are acquired, the coordinates of the probe are transformed into coordinates relative to the reference object, the coordinates relative to reference object transformed from the coordinated of the probe are directly used as a target registration point, and the collection continues to be performed if the pedal is not released.

(5) When the pedal is stepped on, a collection signal is triggered, current coordinates of the probe are collected and accumulated to a specific amount, an average value of the coordinates is calculated, then the average value of the coordinates of the probe is transformed into relative coordinates relative to the reference object to generate a collection point, and the current collection process ends.

In this replacement solution, after the pedal is stepped on, the coordinates of the probe are acquired, after the collection points acquired are accumulated to a specific amount, averaging is performed to the collected points, the average value of the coordinates of the probe is transformed into coordinates relative to the reference object, the coordinates relative to the reference object transformed from the average value of the coordinates of the probe is used as a target registration point, and the collection ends.

(6) When the pedal is stepped on, a collection signal is triggered, current coordinates of the probe are collected and accumulated to a specific amount, an average value of the coordinates is calculated, then the average value of the coordinates of the probe is transformed into relative coordinates relative to the reference object to generate a collection point, and the current collection process is continued till a pedal release signal is received.

In this replacement solution, after the pedal is stepped on, the coordinates of the probe are acquired, after the collection points acquired are accumulated to a specific amount, averaging is performed to the collected points, the average value of the coordinates of the probe is transformed into coordinates relative to the reference object, the coordinates relative to the reference object transformed from the average value of the coordinates of the probe is used as a target registration point, and the collection continues to be performed if the pedal is not released.

(7) When the pedal is stepped on, a collection signal is triggered, current coordinates of the probe are collected, the current coordinates of the probe are transformed into relative coordinates relative to the reference object, after the relative coordinates are accumulated to a specific amount, an average value of the relative coordinates is calculated to generate a collection point, and the current collection process ends.

**In** this replacement solution, after the pedal is stepped on, coordinates of the probe are acquired, after the coordinates of the probe are transformed into coordinates relative to the reference object and the collection points are accumulated to a specific amount, averaging is performed to the collected points, a registration point is generated based on the average value and the relative coordinates relative to the reference object transformed from the coordinates of the probe, and the collection ends.

(8) When the pedal is stepped on, a collection signal is triggered, current coordinates of the probe are collected, the current coordinates of the probe are transformed into relative coordinates relative to the reference object, after the relative coordinates are accumulated to a specific amount, an average value of the relative coordinates is calculated to generate a collection point, and the current collection process is continued till a pedal release signal is received.

**In** this replacement solution, after the pedal is stepped on, coordinates of the probe are acquired, after the coordinates of the probe are transformed into relative coordinates relative to the reference object and the collected points are accumulated to a specific amount, averaging is performed to the collected points, a registration point is generated based on the average value and the relative coordinates relative to the reference object transformed from the coordinates of the probe, and the current collection process is continued till a pedal release signal is received.

Specifically, the foregoing replacement solution may be summarized as shown in the following Table 1.

**Table 1**

| Type of Collected Coordinates | Calculation Method | Generation Method of Registration Point | Duration |
|---|---|---|---|
| Coordinates of the probe | | Registration point is generated | |
| Coordinates of the probe | | Registration point is generated | Collection continues to be performed if the foot pedal is not released |
| Coordinates of the probe relative to the reference frame | | Registration point is generated | |
| Coordinates of the probe relative to the reference frame | | Registration point is generated | Collection continues to be performed if the foot pedal is not released |
| Coordinates of the probe | Cumulative average | Registration point is generated based on the average value | |
| Coordinates of the probe | Cumulative | Registration point is | Collection continues to |
| | average | generated based on the average value | be performed if the foot pedal is not released |
| Coordinates of the probe relative to the reference frame | Cumulative average | Registration point is generated based on the average value | |
| Coordinates of the probe relative to the reference frame | Cumulative average | Registration point is generated based on the average value | Collection continues to be performed if the foot pedal is not released |

Therefore, in some embodiments of the present disclosure, single-point collection may be performed, or multiple-point continuous collection may be performed in the present disclosure. That is, the solution of the present disclosure may be performed once each time a point is collected, or the solution of the present disclosure may be performed once after a plurality of points are continuously collected. This is not limited herein.

In some embodiments of the present disclosure, an embodiment of the present disclosure further provides a registration method. A registration point in the registration method is determined based on the registration point determination method in the foregoing embodiments. For brevity, details are not described herein again.

It should be noted that, in the registration point determination method provided in the embodiments of the present disclosure, the execution body may be a registration point determination apparatus, or a control module, configured to execute the registration point determination method, in the registration point determination apparatus.

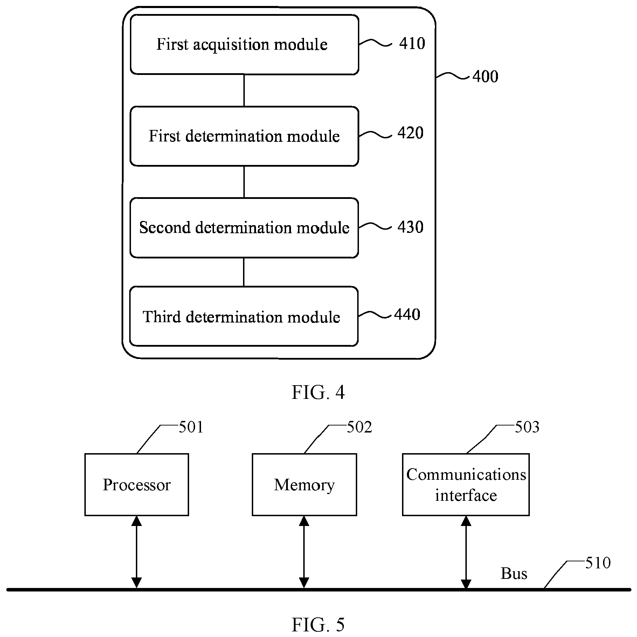

Based on a same inventive concept as the foregoing registration point determination method, the present disclosure further provides a registration point determination apparatus. With reference to FIG. 4, the registration point determination apparatus provided by this embodiment of the present disclosure will be described in detail in the following.

FIG. 4 is a schematic structural diagram of a registration point determination apparatus according to an exemplary embodiment.

As shown in FIG. 4, the registration point determination apparatus 400 may include:
a first acquisition module 410, configured to acquire a first pose data set of a probe when a collection signal is received;
a first determination module 420, configured to determine a hovering coordinate set of the probe based on the first pose data set of the probe; and
a second determination module 430, configured to determine, based on the hovering coordinate set, a registration point corresponding to the first pose data set of the probe.

In this embodiment of the present disclosure, the first pose data set of the probe may be acquired by the first acquisition module 410 when the collection signal is received. The first determination module 420 determines the hovering coordinate set of the probe based on the first pose data set of the probe. The second determination module 430 determines, based on the hovering coordinate set, the registration point corresponding to the first pose data set of the probe. Thus, a set including at least one piece of pose data of the probe, that is, the first pose data set of the probe, is acquired at a time, and then data that meets a hover condition is determined from the first pose data set to determine the hovering coordinate set. Then, data that may be used as the registration point is determined from the first pose data set of the probe based on the hovering coordinate set, which solves a problem that a signal needs to be transmitted during each manually hovering of a probe to collect the point. Therefore, labor is saved to the maximum extent possible while reducing errors caused by human operations, thereby saving time and effort, improving collection speed and convenience, and achieving high collection efficiency.

In some embodiments of the present disclosure, to accurately determine a hover position of the probe, the first acquisition module 410 may be specifically configured to: acquire the first pose data set of the probe and a second pose data set of a reference object when the collection signal is received.

The first determination module 420 may specifically include:
a first determination unit 4201, configured to determine, based on the first pose data set and the second pose data set, a relative coordinate data set of the probe relative to the reference object; and
a second determination unit 4202, configured to determine the hovering coordinate set of the probe based on the relative coordinate data set.

In some embodiments of the present disclosure, to further accurately determine the hover position of the probe, the first determination module 420 may further include:
a third determination unit 4203, configured to determine a check data set based on the first pose data set of the probe; and
a fourth determination unit 4204, configured to determine the hovering coordinate set of the probe based on the first pose data set of the probe and the check data set.

In some embodiments of the present disclosure, to further accurately determine the hover position of the probe, the third determination unit 4203 may be specifically configured to: determine an A^{th} element in the check data set, where the determination is as follows: calculating an average value of top A elements in the first pose data set of the probe, and determining the average value as the A^{th} element in the check data set, to obtain the check data set, where A is a positive integer.

In some embodiments of the present disclosure, to further accurately determine the hover position of the probe, the fourth determination unit 4204 is specifically configured to: determine the first element in the first pose data set of the probe as the first element in the hovering coordinate set of the probe; and determine a B^{th} element in the hovering coordinate set, where the determination is as follows: when a distance between coordinates corresponding to a (B-1)^{th} element in the check data set and coordinates corresponding to a B^{th} element in the first pose data set of the probe meets a hover condition, determining the B^{th} element in the first pose data set of the probe as the B^{th} element in the hovering coordinate set; or when the distance between the coordinates corresponding to a (B-1)^{th} element in the check data set and the coordinates corresponding to a B^{th} element in the first pose data set of the probe the hover condition fails to meet the hover condition, determining that the hovering coordinate set includes only B-1 elements, where B is a positive integer, and B ≥ 2.

In some embodiments of the present disclosure, to accurately determine a registration point, the foregoing registration point determination apparatus may further include:
a third determination module 440, configured to: when a quantity of elements in the hovering coordinate set meets a preset quantity threshold, determine coordinates corresponding to a last element in the hovering coordinate set;
a fourth determination module 450, configured to determine average coordinates of coordinates corresponding to all elements in the hovering coordinate set; and
a fifth determination module 460, configured to: when a distance between the coordinates corresponding to the last element and the average coordinates meets a registration point condition, determine that there is a registration point in the hovering coordinate set.

Correspondingly, the second determination module 430 may be specifically configured to: determine, based on the hovering coordinate set, the registration point corresponding to the first pose data set of the probe when there is a registration point in the hovering coordinate set.

In some embodiments of the present disclosure, to accurately determine the registration point, the second determination module 430 may be specifically configured to: determine, based on the hovering coordinate set, average coordinates of the coordinates corresponding to all elements in the hovering coordinate set; and determine a point corresponding to the average coordinates of the coordinates corresponding to all elements in the hovering coordinate set as the registration point corresponding to the first pose data set of the probe.

In some embodiments of the present disclosure, the first acquisition module 410 may be specifically configured to: acquire the first pose data set of the probe with in a preset time period when the collection signal is received.

In some embodiments of the present disclosure, the first acquisition module 410 may be specifically configured to: acquire the first pose data set of the probe during a period from a start to an end of the collection signal when the collection signal is received.

In some embodiments of the present disclosure, the first pose data set includes a first data enable bit, a first data frame sequence, and first time stamp information, and the second pose data set includes a second data enable bit, a second data frame sequence, and second time stamp information.

The first determination unit may be specifically configured to: determine, based on the first pose data set and the second pose data set, the relative coordinate data set of the probe relative to the reference object when the first data enable bit and the second data enable bit are preset data enable bits, the first data frame sequence is the same as the second data frame sequence, and the first time stamp information is the same as the second time stamp information.

The registration point determination apparatus provided in this embodiment of the present disclosure may be configured to execute the registration point determination method provided in the foregoing method embodiments. Implementation principles and technical effects thereof are similar. For brevity, details are not described herein again.

Based on a same inventive concept, an embodiment of the present disclosure further provides an electronic device.

FIG. 5 is a schematic structural diagram of an electronic device according to an embodiment of the present disclosure. As shown in FIG. 5, the electronic device may include a processor 501 and a memory 502 storing a computer program or instructions.

Specifically, the processor 501 may include a central processing unit (CPU), an application-specific integrated circuit (Application Specific Integrated Circuit, ASIC), or one or more integrated circuits that may be configured to implement the embodiments of the present disclosure.

The memory 502 may include a mass memory for data or instructions. By way of example without limitation, the memory 502 may include a hard disk drive (Hard Disk Drive, HDD), a floppy disk drive, a flash memory, an optical disc, a magneto-optical disc, a magnetic tape, a universal serial bus (Universal Serial Bus, USB) drive, or a combination of two or more thereof. When appropriate, the memory 502 may include a removable or non-removable (or fixed) medium. When appropriate, the memory 502 may be internal or external to an integrated gateway disaster recovery device. In a particular embodiment, the memory 502 is a non-volatile solid state memory. The memory may include a read-only memory (Read Only Memory image, ROM), a random access memory (Random-Access Memory, RAM), a magnetic disk storage medium device, an optical storage medium device, a flash device, or an electrical, optical, or another physical/tangible memory storage device. Therefore, the memory generally includes one or more tangible (non-transient) computer-readable storage media (for example, memory devices) encoded with software including computer executable instructions, and the software, when executed (for example, by one or a plurality of processors), is operable to perform the operations described in the registration point determination method provided in the foregoing embodiments.

The processor 501 reads and executes the computer program instructions stored in the memory 502, to implement any one of the registration point determination methods in the foregoing embodiments.

In an example, the electronic device may further include a communications interface 503 and a bus 510. As shown in FIG. 5, the processor 501, the memory 502, and the communications interface 503 are connected through the bus 510 to communicate with each other.

The communications interface 503 is mainly configured to implement communication between the modules, devices, units, and/or devices in the embodiments of the present disclosure.

The bus 510 includes hardware, software, or both, and components of the electronic device are coupled to each other. By way of example without limitation, the bus may include an accelerated graphics port (AGP) or other graphics buses, an enhanced industry standard architecture (EISA) bus, a front side bus (FSB), a hypertransport (HT) interconnect bus, an industry standard architecture (ISA) bus, an infinite bandwidth interconnect bus, a low pin count (LPC) bus, a memory bus, a micro channel architecture (MCA) bus, a peripheral component interconnect (PCI) bus, a PCI-Express (PCI-X) bus, a serial advanced technology attachment (SATA) bus, a video electronics standards association local bus (VLB) or other suitable buses, or a combination of two or more thereof. When appropriate, the bus 510 may include one or more buses. Although the specific buses are described and illustrated in the embodiments of the present disclosure, any suitable bus or interconnect is contemplated in the present disclosure.

The electronic device may execute the registration point determination method in the embodiments of the present disclosure, so as to implement the registration point determination method illustrated in FIG. 1 or FIG. 3.

In addition, an embodiment of the present disclosure may provide a readable storage medium to implement the registration point determination method in the foregoing embodiments. The readable storage medium has stored thereon program instructions that, when executed by a processor, cause any one of the registration point determination methods in the foregoing embodiments to be implemented.

In addition, an embodiment of the present disclosure may provide a computer program product to implement the registration point determination method in the foregoing embodiments. An instruction in the computer program product, when executed by a processor of an electronic device, causes the electronic device to execute any one of the registration point determination methods in the foregoing embodiments.

It should be made clear that the present disclosure is not limited to the specific configuration and processing described above and shown in the figures. For brevity, a detailed description of a known method is omitted herein. In the foregoing embodiments, several specific steps are described and shown as examples. However, the method process in the present disclosure is not limited to the specific steps described and shown.

The functional blocks shown in the foregoing structural block diagram may be implemented as hardware, software, firmware, or a combination thereof. When the functional blocks are implemented as hardware, the functional blocks may be, for example, an electronic circuit, an disclosure-specific integrated circuit (ASIC), appropriate firmware, a plug-in, or a function card. When the functional blocks are implemented as software, the elements of the present disclosure are programs or code segments used to perform required tasks. The program or code segment may be stored in a machine-readable medium, or transmitted on a transmission medium or a communication link through a data signal carried in a carrier wave. "Machine-readable medium" may include any medium that can store or transmit information. Examples of machine-readable media include an electronic circuit, a semiconductor memory device, a ROM, a flash memory, an erasable ROM (EROM), a floppy disk, a CD-ROM, an optical disk, a hard disk, a fiber optic medium, a radio frequency (RF) link, and the like. The code segment may be downloaded via a computer network such as the Internet, intranet, and the like.

Various aspects of the present disclosure are described above with reference to the flowcharts and/or block diagrams of the methods, apparatuses (systems), and computer program products according to the embodiments of the present disclosure. It should be understood that, each procedure and/or block in the flowchart and/or the block diagram as well as a combination of procedures and/or blocks in the flowchart and/or the block diagram may be implemented by computer program instructions. These computer program instructions may be provided for a general-purpose computer, a dedicated computer, or a processor of another programmable data processing apparatus to generate a machine, so that when these instructions are executed by the computer or the processor of the another programmable data processing apparatus, specific functions/actions in one or more blocks in the flowcharts and/or in the block diagrams are implemented. The processor may be but is not limited to a general purpose processor, a dedicated processor, a special application processor, or a field programmable logic circuit. It may also be understood that, each block and/or procedure in the block diagram and/or the flowchart as well as a combination of blocks and/or procedures in the block diagram and/or the flowchart may alternatively be implemented by special-purpose hardware for executing a specific function or action, or may be implemented by a combination of special-purpose hardware and computer instructions.

The foregoing descriptions are merely specific implementations of the present disclosure. It may be clearly understood by a person skilled in the art that, for the purpose of convenient and brief description, for a detailed working process of the foregoing system, module, and unit, reference may be made to corresponding processes in the foregoing method embodiments, and details are not described herein again.

## Claims

1. A computer-implemented registration point determination method, comprising
acquiring (S110) a first pose data set of a probe when a collection signal is received;
determining (S120), based on the first pose data set of the probe, a hovering coordinate set of the probe, wherein the hovering coordinate set is a set of coordinate data of the probe that meets a hover condition, wherein the hover condition is a preset condition for determining that the probe is in a hover state; and
determining (S130), based on the hovering coordinate set, a registration point corresponding to the first pose data set of the probe;
wherein the determining (S130), based on the hovering coordinate set, a registration point corresponding to the first pose data set of the probe, comprises:
determining (S1303), based on the hovering coordinate set, average coordinates of coordinates corresponding to all elements in the hovering coordinate set; and
determining (S1304) a point corresponding to the average coordinates of the coordinates corresponding to all elements in the hovering coordinate set as the registration point corresponding to the first pose data set of the probe.

2. The method according to claim 1, wherein the acquiring (S110) a first pose data set of a probe when a collection signal is received comprises:
acquiring (S1103) the first pose data set of the probe and a second pose data set of a reference object when the collection signal is received,
wherein the determining (S 120), based on the first pose data set of the probe, a hovering coordinate set of the probe comprises:
determining (S12031), based on the first pose data set and the second pose data set, a relative coordinate data set of the probe relative to the reference object; and
determining (S12032), based on the relative coordinate data set, the hovering coordinate set of the probe.

3. The method according to claim 1, wherein the determining (S120) a hovering coordinate set of the probe based on the first pose data set of the probe comprises:
determining (S 1201), based on the first pose data set of the probe, a check data set; and
determining (S 1202), based on the first pose data set of the probe and the check data set, the hovering coordinate set of the probe.

4. The method according to claim 3, wherein the determining (S1201), based on the first pose data set of the probe, a check data set comprises:
determining (S12011) an A^{th} element in the check data set, wherein the determination is as follows:
calculating an average value of top A elements in the first pose data set of the probe, and determining that the average value is the A^{th} element in the check data set to obtain the check data set,
wherein A is a positive integer.

5. The method according to claim 3, wherein the determining (S1202) the hovering coordinate set of the probe based on the first pose data set of the probe and the check data set comprises:
determining (S12021) a first element in the first pose data set of the probe as a first element in the hovering coordinate set of the probe; and
determining (S12022) a B^{th} element in the hovering coordinate set, wherein the determination is as follows: in a case where a distance between coordinates corresponding to a (B-1)^{th} element in the check data set and coordinates corresponding to a B^{th} element in the first pose data set of the probe meets a hover condition, determining the B^{th} element in the first pose data set of the probe as the B^{th} element in the hovering coordinate set; and in a case where the distance between coordinates corresponding to a (B-1)^{th} element in the check data set and coordinates corresponding to a B^{th} element in the first pose data set of the probe fails to meet the hover condition, determining that the hovering coordinate set comprises only B-1 elements, wherein B is a positive integer, and B ≥ 2.

6. The method according to claim 1, wherein before the determining (S130), based on the hovering coordinate set, a registration point corresponding to the first pose data set of the probe, the method further comprises:
determining (S125) coordinates corresponding to a last element in the hovering coordinate set when a quantity of elements in the hovering coordinate set meets a preset quantity threshold;
determining (S126) the average coordinates of coordinates corresponding to all elements in the hovering coordinate set; and
determining (S127) that there is a registration point in the hovering coordinate set when a distance between the coordinates corresponding to the last element and the average coordinates meets a registration point condition; and
the determining (S130), based on the hovering coordinate set, a registration point corresponding to the first pose data set of the probe further comprises:
determining (S1301), based on the hovering coordinate set, the registration point corresponding to the first pose data set of the probe when there is a registration point in the hovering coordinate set.

7. The method according to claim 1, wherein the acquiring (S110) a first pose data set of a probe when a collection signal is received comprises:
acquiring (S1102) the first pose data set of the probe within a preset time period when the collection signal is received.

8. The method according to claim 1, wherein the acquiring (S110) a first pose data set of a probe when a collection signal is received comprises:
acquiring (S1101) the first pose data set of the probe during a period from a start to an end of the collection signal when the collection signal is received.

9. The method according to claim 2, wherein the first pose data set comprises a first data enable bit, a first data frame sequence, and first time stamp information, and the second pose data set comprises a second data enable bit, a second data frame sequence, and second time stamp information; and
the determining (S12031), based on the first pose data set and the second pose data set, a relative coordinate data set of the probe relative to the reference object comprises:
determining (S120311), based on the first pose data set and the second pose data set, the relative coordinate data set of the probe relative to the reference object when the first data enable bit and the second data enable bit are preset data enable bits, the first data frame sequence is the same as the second data frame sequence, and the first time stamp information is the same as the second time stamp information.

10. A registration method, wherein a registration point in the registration method is determined based on the registration point determination method according to any one of claims 1 to 9.

11. A registration point determination apparatus, comprising:
a first acquisition module (410), configured to acquire a first pose data set of a probe when a collection signal is received;
a first determination module (420), configured to determine a hovering coordinate set of the probe based on the first pose data set of the probe, wherein the hovering coordinate set is a set of coordinate data of the probe that meets a hover condition, wherein the hover condition is a preset condition for determining that the probe is in a hover state ; and
a second determination module (430), configured to determine, based on the hovering coordinate set, a registration point corresponding to the first pose data set of the probe; wherein the second determination module (430) is further configured to:
determine, based on the hovering coordinate set, average coordinates of coordinates corresponding to all elements in the hovering coordinate set; and
determine a point corresponding to the average coordinates of the coordinates corresponding to all elements in the hovering coordinate set as the registration point corresponding to the first pose data set of the probe.

12. An electronic device, comprising a processor (501) and a memory (502), wherein the memory stores computer program instructions, and when the computer program instructions are executed by the processor, the processor is configured to implement the registration point determination method according to any one of claims 1 to 9.

13. A computer-readable storage medium, storing computer program instructions, wherein when the computer program instructions are executed a processor, the registration point determination method according to any one of claims 1 to 9 is implemented.

14. A computer program product, wherein when instructions in the computer program product are executed by a processor of an electronic device, the electronic device is configured to implement the registration point determination method according to any one of claims 1 to 9.

## Patentansprüche

1. Computerimplementiertes Verfahren zur Bestimmung eines Registrierungspunkts, umfassend:
Erfassen (S110) eines ersten Posendatensatzes einer Sonde, wenn ein Erfassungssignal empfangen wird;
Bestimmen (S120), basierend auf dem ersten Posendatensatz der Sonde, eines Schwebekoordinatensatzes der Sonde, wobei der Schwebekoordinatensatz ein Satz von Koordinatendaten der Sonde ist, die eine Schwebebedingung erfüllen, wobei die Schwebebedingung eine vorgegebene Bedingung zum Bestimmen ist, dass sich die Sonde in einem Schwebezustand befindet; und
Bestimmen (S130), basierend auf dem Schwebekoordinatensatz, eines dem ersten Posendatensatz der Sonde entsprechenden Registrierungspunkts;
wobei das Bestimmen (S130), basierend auf dem Schwebekoordinatensatz, eines dem ersten Posendatensatz der Sonde entsprechenden Registrierungspunkts umfasst:
Bestimmen (S1303), basierend auf dem Schwebekoordinatensatz, von Durchschnittskoordinaten der Koordinaten, die allen Elementen in dem Schwebekoordinatensatz entsprechen; und
Bestimmen (S1304) eines den Durchschnittskoordinaten der Koordinaten, die allen Elementen in dem Schwebekoordinatensatz entsprechen, entsprechenden Punkts als den dem ersten Posendatensatz der Sonde entsprechenden Registrierungspunkt.

2. Verfahren nach Anspruch 1, wobei das Erfassen (S110) eines ersten Posendatensatzes einer Sonde, wenn ein Erfassungssignal empfangen wird, umfasst:
Erfassen (S1103) des ersten Posendatensatzes der Sonde und eines zweiten Posendatensatzes eines Referenzobjekts, wenn das Erfassungssignal empfangen wird,
wobei das Bestimmen (S120), basierend auf dem ersten Posendatensatz der Sonde, eines Schwebekoordinatensatzes der Sonde umfasst:
Bestimmen (S12031), basierend auf dem ersten Posendatensatz und dem zweiten Posendatensatz, eines Relativkoordinatendatensatzes der Sonde relativ zu dem Referenzobjekt; und
Bestimmen (S12032), basierend auf dem Relativkoordinatendatensatz, des Schwebekoordinatensatzes der Sonde.

3. Verfahren nach Anspruch 1, wobei das Bestimmen (S120) eines Schwebekoordinatensatzes der Sonde basierend auf dem ersten Posendatensatz der Sonde umfasst:
Bestimmen (S1201), basierend auf dem ersten Posendatensatz der Sonde, eines Prüfdatensatzes; und
Bestimmen (S1202), basierend auf dem ersten Posendatensatz der Sonde und dem Prüfdatensatz, des Schwebekoordinatensatzes der Sonde.

4. Verfahren nach Anspruch 3, wobei das Bestimmen (S1201), basierend auf dem ersten Posendatensatz der Sonde, eines Prüfdatensatzes umfasst:
Bestimmen (S12011) eines A-ten Elements in dem Prüfdatensatz, wobei die Bestimmung wie folgt erfolgt:
Berechnen eines Mittelwerts der ersten A Elemente in dem ersten Posendatensatz der Sonde und Bestimmen des Mittelwerts als das A-te Element in dem Prüfdatensatz, um den Prüfdatensatz zu erhalten,
wobei A eine positive ganze Zahl ist.

5. Verfahren nach Anspruch 3, wobei das Bestimmen (S1202) des Schwebekoordinatensatzes der Sonde basierend auf dem ersten Posendatensatz der Sonde und dem Prüfdatensatz umfasst:
Bestimmen (S12021) eines ersten Elements in dem ersten Posendatensatz der Sonde als ein erstes Element in dem Schwebekoordinatensatz der Sonde; und
Bestimmen (S12022) eines B-ten Elements in dem Schwebekoordinatensatz, wobei die Bestimmung wie folgt erfolgt:
wenn ein Abstand zwischen Koordinaten, die einem (B-1)-ten Element in dem Prüfdatensatz entsprechen, und Koordinaten, die einem B-ten Element in dem ersten Posendatensatz der Sonde entsprechen, eine Schwebebedingung erfüllt, Bestimmen des B-ten Elements in dem ersten Posendatensatz der Sonde als das B-te Element in dem Schwebekoordinatensatz; und
wenn der Abstand zwischen den Koordinaten, die dem (B-1)-ten Element in dem Prüfdatensatz entsprechen, und den Koordinaten, die dem B-ten Element in dem ersten Posendatensatz der Sonde entsprechen, die Schwebebedingung nicht erfüllt, Bestimmen, dass der Schwebekoordinatensatz nur B-1 Elemente umfasst,
wobei B eine positive ganze Zahl ist und B ≥ 2 gilt.

6. Verfahren nach Anspruch 1, wobei das Verfahren vor dem Bestimmen (S130), basierend auf dem Schwebekoordinatensatz, eines dem ersten Posendatensatz der Sonde entsprechenden Registrierungspunkts ferner umfasst:
Bestimmen (S125) von Koordinaten, die einem letzten Element in dem Schwebekoordinatensatz entsprechen, wenn eine Anzahl von Elementen in dem Schwebekoordinatensatz einen vorgegebenen Anzahlsschwellenwert erfüllt;
Bestimmen (S126) der Durchschnittskoordinaten der Koordinaten, die allen Elementen in dem Schwebekoordinatensatz entsprechen; und
Bestimmen (S127), dass ein Registrierungspunkt in dem Schwebekoordinatensatz vorhanden ist, wenn ein Abstand zwischen den dem letzten Element entsprechenden Koordinaten und den Durchschnittskoordinaten eine Registrierungspunktbedingung erfüllt; und
wobei das Bestimmen (S130), basierend auf dem Schwebekoordinatensatz, eines dem ersten Posendatensatz der Sonde entsprechenden Registrierungspunkts ferner umfasst:
Bestimmen (S1301), basierend auf dem Schwebekoordinatensatz, des dem ersten Posendatensatz der Sonde entsprechenden Registrierungspunkts, wenn ein Registrierungspunkt in dem Schwebekoordinatensatz vorhanden ist.

7. Verfahren nach Anspruch 1, wobei das Erfassen (S110) eines ersten Posendatensatzes einer Sonde, wenn ein Erfassungssignal empfangen wird, umfasst:
Erfassen (S1102) des ersten Posendatensatzes der Sonde innerhalb eines vorgegebenen Zeitraums, wenn das Erfassungssignal empfangen wird.

8. Verfahren nach Anspruch 1, wobei das Erfassen (S110) eines ersten Posendatensatzes einer Sonde, wenn ein Erfassungssignal empfangen wird, umfasst:
Erfassen (S1101) des ersten Posendatensatzes der Sonde während eines Zeitraums von einem Beginn bis zu einem Ende des Erfassungssignals, wenn das Erfassungssignal empfangen wird.

9. Verfahren nach Anspruch 2, wobei der erste Posendatensatz ein erstes Datenfreigabebit, eine erste Datenrahmensequenz und erste Zeitstempelinformationen umfasst und der zweite Posendatensatz ein zweites Datenfreigabebit, eine zweite Datenrahmensequenz und zweite Zeitstempelinformationen umfasst; und
wobei das Bestimmen (S12031), basierend auf dem ersten Posendatensatz und dem zweiten Posendatensatz, eines Relativkoordinatendatensatzes der Sonde relativ zu dem Referenzobjekt umfasst:
Bestimmen (S120311), basierend auf dem ersten Posendatensatz und dem zweiten Posendatensatz, des Relativkoordinatendatensatzes der Sonde relativ zu dem Referenzobjekt,
wenn das erste Datenfreigabebit und das zweite Datenfreigabebit vorgegebene Datenfreigabebits sind, die erste Datenrahmensequenz mit der zweiten Datenrahmensequenz übereinstimmt und die ersten Zeitstempelinformationen mit den zweiten Zeitstempelinformationen übereinstimmen.

10. Registrierungsverfahren, wobei ein Registrierungspunkt in dem Registrierungsverfahren basierend auf dem Verfahren zur Bestimmung eines Registrierungspunkts nach einem der Ansprüche 1 bis 9 bestimmt wird.

11. Vorrichtung zur Bestimmung eines Registrierungspunkts, umfassend:
ein erstes Erfassungsmodul (410), das dazu eingerichtet ist, einen ersten Posendatensatz einer Sonde zu erfassen, wenn ein Erfassungssignal empfangen wird;
ein erstes Bestimmungsmodul (420), das dazu eingerichtet ist, basierend auf dem ersten Posendatensatz der Sonde einen Schwebekoordinatensatz der Sonde zu bestimmen, wobei der Schwebekoordinatensatz ein Satz von Koordinatendaten der Sonde ist, die eine Schwebebedingung erfüllen, wobei die Schwebebedingung eine vorgegebene Bedingung zum Bestimmen ist, dass sich die Sonde in einem Schwebezustand befindet; und
ein zweites Bestimmungsmodul (430), das dazu eingerichtet ist, basierend auf dem Schwebekoordinatensatz einen dem ersten Posendatensatz der Sonde entsprechenden Registrierungspunkt zu bestimmen;
wobei das zweite Bestimmungsmodul (430) ferner dazu eingerichtet ist:
basierend auf dem Schwebekoordinatensatz Durchschnittskoordinaten der Koordinaten zu bestimmen, die allen Elementen in dem Schwebekoordinatensatz entsprechen; und
einen den Durchschnittskoordinaten der Koordinaten, die allen Elementen in dem Schwebekoordinatensatz entsprechen, entsprechenden Punkt als den dem ersten Posendatensatz der Sonde entsprechenden Registrierungspunkt zu bestimmen.

12. Elektronische Vorrichtung, umfassend einen Prozessor (501) und einen Speicher (502), wobei der Speicher Computerprogrammanweisungen speichert und der Prozessor, wenn die Computerprogrammanweisungen durch den Prozessor ausgeführt werden, dazu eingerichtet ist, das Verfahren zur Bestimmung eines Registrierungspunkts nach einem der Ansprüche 1 bis 9 auszuführen.

13. Computerlesbares Speichermedium, auf dem Computerprogrammanweisungen gespeichert sind, wobei, wenn die Computerprogrammanweisungen von einem Prozessor ausgeführt werden, das Verfahren zur Bestimmung eines Registrierungspunkts nach einem der Ansprüche 1 bis 9 ausgeführt wird.

14. Computerprogrammprodukt, wobei, wenn Anweisungen in dem Computerprogrammprodukt von einem Prozessor einer elektronischen Vorrichtung ausgeführt werden, die elektronische Vorrichtung dazu eingerichtet ist, das Verfahren zur Bestimmung eines Registrierungspunkts nach einem der Ansprüche 1 bis 9 auszuführen.

## Revendications

1. Procédé de détermination d'un point d'enregistrement mise en œuvre par ordinateur, comprenant :
acquérir (S110) un premier ensemble de données de pose d'une sonde lorsqu'un signal de collecte est reçu ;
déterminer (S120), sur la base du premier ensemble de données de pose de la sonde, un ensemble de coordonnées en suspension de la sonde, l'ensemble de coordonnées en suspension étant un ensemble de données de coordonnées de la sonde qui satisfait à une condition de suspension, la condition de suspension étant une condition prédéfinie pour déterminer que la sonde est dans un état de suspension ; et
déterminer (S130), sur la base de l'ensemble de coordonnées en suspension, un point d'enregistrement correspondant au premier ensemble de données de pose de la sonde ;
dans lequel la détermination (S130) d'un point d'enregistrement correspondant au premier ensemble de données de pose de la sonde sur la base de l'ensemble de coordonnées en suspension, comprend :
déterminer (S1303), sur la base de l'ensemble de coordonnées en suspension, des coordonnées moyennes des coordonnées correspondant à tous les éléments de l'ensemble de coordonnées en suspension ; et
déterminer (S1304) un point correspondant aux coordonnées moyennes des coordonnées correspondant à tous les éléments de l'ensemble de coordonnées en suspension comme point d'enregistrement correspondant au premier ensemble de données de pose de la sonde.

2. Procédé selon la revendication 1, dans lequel l'acquisition (S110) d'un premier ensemble de données de pose d'une sonde lorsqu'un signal de collecte est reçu comprend :
acquérir (S1103) le premier ensemble de données de pose de la sonde et un second ensemble de données de pose d'un objet de référence lorsque le signal de collecte est reçu,
dans lequel, la détermination (S120) d'un ensemble de coordonnées en suspension de la sonde sur la base du premier ensemble de données de pose de la sonde, comprend :
déterminer (S12031), sur la base du premier ensemble de données de pose et du second ensemble de données de pose, un ensemble de données de coordonnées relatives de la sonde par rapport à l'objet de référence ; et
déterminer (S12032), sur la base de l'ensemble de données de coordonnées relatives, l'ensemble de coordonnées en suspension de la sonde.

3. Procédé selon la revendication 1, dans lequel la détermination (S120) d'un ensemble de coordonnées en suspension de la sonde sur la base du premier ensemble de données de pose de la sonde, comprend :
déterminer (S1201), sur la base du premier ensemble de données de pose de la sonde, un ensemble de données de vérification ; et
déterminer (S1202), sur la base du premier ensemble de données de pose de la sonde et de l'ensemble de données de vérification, l'ensemble de coordonnées en suspension de la sonde.

4. Procédé selon la revendication 3, dans lequel la détermination (S1201) d'un ensemble de données de vérification sur la base du premier ensemble de données de pose de la sonde comprend :
déterminer (S12011) un A-ème élément de l'ensemble de données de vérification, la détermination étant comme suit :
calculer une valeur moyenne de A premiers éléments dans le premier ensemble de données de pose de la sonde, et déterminer que la valeur moyenne est le A-ème élément de l'ensemble de données de vérification pour obtenir l'ensemble de données de vérification,
où A est un entier positif.

5. Procédé selon la revendication 3, dans lequel la détermination (S1202) de l'ensemble de coordonnées en suspension de la sonde sur la base du premier ensemble de données de pose de la sonde et de l'ensemble de données de vérification, comprend :
déterminer (S12021) un premier élément dans le premier ensemble de données de pose de la sonde comme premier élément de l'ensemble de coordonnées en suspension de la sonde ; et
déterminer (S12022) un B-ème élément de l'ensemble de coordonnées en suspension, la détermination étant comme suit : dans le cas où une distance entre des coordonnées correspondant à un (B-1)-ème élément de l'ensemble de données de vérification et des coordonnées correspondant à un B-ème élément dans le premier ensemble de données de pose de la sonde satisfait à une condition de suspension, déterminer le B-ème élément dans le premier ensemble de données de pose de la sonde comme B-ème élément de l'ensemble de coordonnées en suspension ; et dans le cas où la distance entre des coordonnées correspondant à un (B-1)-ème élément de l'ensemble de données de vérification et des coordonnées correspondant à un B-ème élément dans le premier ensemble de données de pose de la sonde ne satisfait pas à la condition de suspension, déterminer que l'ensemble de coordonnées en suspension ne comprend que B-1 éléments, où B est un entier positif, et B ≥ 2.

6. Procédé selon la revendication 1, dans lequel avant la détermination (S130) d'un point d'enregistrement correspondant au premier ensemble de données de pose de la sonde sur la base de l'ensemble de coordonnées en suspension, comprend en outre :
déterminer (S125) des coordonnées correspondant à un dernier élément de l'ensemble de coordonnées en suspension lorsqu'un nombre d'éléments de l'ensemble de coordonnées en suspension atteint un seuil de nombre prédéfini ;
déterminer (S126) les coordonnées moyennes des coordonnées correspondant à tous les éléments de l'ensemble de coordonnées en suspension ; et
déterminer (S127) qu'il existe un point d'enregistrement dans l'ensemble de coordonnées en suspension lorsqu'une distance entre les coordonnées correspondant au dernier élément et les coordonnées moyennes satisfait à une condition de point d'enregistrement ; et
dans lequel la détermination (S130) d'un point d'enregistrement correspondant au premier ensemble de données de pose de la sonde sur la base de l'ensemble de coordonnées en suspension, comprend en outre :
déterminer (S1301), sur la base de l'ensemble de coordonnées en suspension, le point d'enregistrement correspondant au premier ensemble de données de pose de la sonde lorsqu'il existe un point d'enregistrement dans l'ensemble de coordonnées en suspension.

7. Procédé selon la revendication 1, dans lequel l'acquisition (S110) d'un premier ensemble de données de pose d'une sonde lorsqu'un signal de collecte est reçu comprend :
acquérir (S1102) le premier ensemble de données de pose de la sonde dans une durée prédéfinie lorsque le signal de collecte est reçu.

8. Procédé selon la revendication 1, dans lequel l'acquisition (S110) d'un premier ensemble de données de pose d'une sonde lorsqu'un signal de collecte est reçu comprend :
acquérir (S1101) le premier ensemble de données de pose de la sonde pendant une durée allant d'un début à une fin du signal de collecte lorsque le signal de collecte est reçu.

9. Procédé selon la revendication 2, dans laquelle le premier ensemble de données de pose comprend un premier bit d'activation de données, une première séquence de trame de données et une première information d'horodatage, et le second ensemble de données de pose comprend un second bit d'activation de données, une seconde séquence de trame de données et une seconde information d'horodatage ; et
la détermination (S12031) d'un ensemble de données de coordonnées relatives de la sonde par rapport à l'objet de référence sur la base du premier ensemble de données de pose et du second ensemble de données de pose, comprend :
déterminer (S120311), sur la base du premier ensemble de données de pose et du second ensemble de données de pose, l'ensemble de données de coordonnées relatives de la sonde par rapport à l'objet de référence lorsque le premier bit d'activation de données et le second bit d'activation de données sont des bits d'activation de données prédéfinis, que la première séquence de trame de données est la même que la seconde séquence de trame de données, et que la première information d'horodatage est la même que la seconde information d'horodatage.

10. Procédé d'enregistrement, dans lequel un point d'enregistrement dans le procédé d'enregistrement est déterminé sur la base du procédé de détermination d'un point d'enregistrement selon l'une quelconque des revendications 1 à 9.

11. Appareil de détermination d'un point d'enregistrement comprenant :
un premier module d'acquisition (410), configuré pour acquérir un premier ensemble de données de pose d'une sonde lorsqu'un signal de collecte est reçu ;
un premier module de détermination (420), configuré pour déterminer un ensemble de coordonnées en suspension de la sonde, sur la base du premier ensemble de données de pose de la sonde, l'ensemble de coordonnées en suspension étant un ensemble de données de coordonnées de la sonde qui satisfait à une condition de suspension, la condition de suspension étant une condition prédéfinie pour déterminer que la sonde est dans un état de suspension ; et
un second module de détermination (430), configuré pour déterminer, sur la base de l'ensemble de coordonnées en suspension, un point d'enregistrement correspondant au premier ensemble de données de pose ; le second module de détermination (430) étant configuré pour :
déterminer, sur la base de l'ensemble de coordonnées en suspension, des coordonnées moyennes des coordonnées correspondant à tous les éléments de l'ensemble de coordonnées en suspension ; et
déterminer un point correspondant aux coordonnées moyennes des coordonnées correspondant à tous les éléments de l'ensemble de coordonnées en suspension comme point d'enregistrement correspondant au premier ensemble de données de pose de la sonde.

12. Dispositif électronique, comprenant un processeur (501) et une mémoire (502), dans lequel la mémoire stocke des instructions de programme informatique, et lorsque les instructions de programme informatique sont exécutées par le processeur, le processeur est configuré pour mettre en œuvre le procédé de détermination d'un point d'enregistrement selon l'une quelconque des revendications 1 à 9.

13. Support de stockage lisible par ordinateur, stockant des instructions de programme informatique, dans lequel lorsque les instructions de programme informatique sont exécutées par un processeur, le procédé de détermination d'un point d'enregistrement selon l'une quelconque des revendications 1 à 9 est mise en œuvre.

14. Produit de programme informatique, dans lequel lorsque des instructions dans le produit de programme informatique sont exécutées par un processeur d'un dispositif électronique, le dispositif électronique est configuré pour mettre en œuvre le procédé de détermination d'un point d'enregistrement selon l'une quelconque des revendications 1 à 9.
